(19) **Europäisches Patentamt / European Patent Office / Office européen des brevets**

(11) **EP 2 502 635 B1**

(12) **EUROPEAN PATENT SPECIFICATION**

(45) Date of publication and mention
of the grant of the patent:
**11.12.2013 Bulletin 2013/50**

(51) Int Cl.:
*A61K 45/06* (2006.01)       *A61P 15/08* (2006.01)
*A23L 1/302* (2006.01)       *A23L 1/305* (2006.01)
*A61K 31/07* (2006.01)       *A61K 31/122* (2006.01)
*A61K 31/198* (2006.01)      *A61K 31/205* (2006.01)
*A61K 31/355* (2006.01)      *A61K 31/375* (2006.01)
*A61K 33/30* (2006.01)       *A61K 31/4415* (2006.01)

(21) Application number: **12172324.1**

(22) Date of filing: **18.06.2008**

(54) **Combined formulations for the treatment of male infertility**

Kombinierte Formulierungen zur Behandlung der männlichen Unfruchtbarkeit

Formulations combinées pour le traitement de la stérilité masculine

(84) Designated Contracting States:
**AT BE BG CH CY CZ DE DK EE ES FI FR GB GR HR HU IE IS IT LI LT LU LV MC MT NL NO PL PT RO SE SI SK TR**

(43) Date of publication of application:
**26.09.2012 Bulletin 2012/39**

(62) Document number(s) of the earlier application(s) in accordance with Art. 76 EPC:
**08425428.3 / 2 135 621**

(73) Proprietor: **Fasani, Roberto**
**24100 Bergamo (IT)**

(72) Inventor: **Fasani, Roberto**
**24100 Bergamo (IT)**

(74) Representative: **Pistolesi, Roberto et al**
**Dragotti & Associati Srl**
**Via Nino Bixio, 7**
**20129 Milano (IT)**

(56) References cited:
WO-A-03/032751       WO-A-03/086080
WO-A-2007/087667    US-A1- 2004 001 817

- **NIEDERBERGER ET AL: "Antioxidant Intake is Associated With Semen Quality in Healthy Men", JOURNAL OF UROLOGY, BALTIMORE, MD, US, vol. 174, no. 6, 1 December 2005 (2005-12-01), page 2301, XP005384177, ISSN: 0022-5347**

Remarks:
The file contains technical information submitted after the application was filed and not included in this specification

**Description**

The present invention refers to a formulation composed of two different formulations to be administered separately, one containing arginine vitamin A, vitamin E, vitamin B6, vitamin B9 (i.e. folic acid), vitamin B12, coenzyme Q10 and the other containing carnitine, arginine, vitamin C and zinc, for treating male infertility.

**DESCRIPTION**

Poor sperm quality not linked to ascertainable causes, the so-called idiopathic oligoasthenoteratozoospermia (the OAT syndrome), is responsible for 40-75% of male infertility cases. Up to date, various pharmacological treatments have been proposed for the OAT syndrome. However, as of date, a rigorous evaluation of the studies available in literature has recently led the authors of *EAU Guidelines on Infertility - 2007,* to the conclusion that the treatments currently known do not meet the therapeutic needs towards such pathology.

For example, as a matter of fact, studies carried out on GnRH show contradictory results while the ones carried out on FSH proved inefficient. In addition, the "kininogene-knin" system activating drugs have not yet been proved efficient while the mast cell blockers and the antiestrogens show a very low efficiency. Lastly, the hCG/hMG combination, the androgens, the bromocriptines and the alpha blockers are not deemed recommendable unless in cases of hypogonadism, in that they cause adverse reactions.

Furthermore, over the last years several studies have shown the importance of the ROS (reactive oxygen species) in the physiopathology of reproductions: as a matter of fact, it has been shown how the oxidative stress combined with high levels of ROS in the seminal fluid (and in particular, but not solely, during inflammatory states leading to a large number of leukocytes) causes a reduction of the motility of sperms and damage to the cellular DNA *(Agarwal et al, 2006; Agarwal et al, 2002).*

Sperms are very sensitive to the ROS given that their cellular membrane contains polyunsaturated fatty acid (PUFA) which are easily subjected to a peroxidation process *(Fraczek et al, 2007; Agarwal et al, 2005).* Thus, the cellular damage would occur when the ROS production exceeds the activity of antioxidant mechanisms usually present in the seminal plasma *(Sheweita et al, 2005).* Various studies have been carried out over the last years regarding various compounds with antioxidant and spermatogenesis adjuvant action in infertile subjects allowing to draw important conclusions regarding the antioxidants:

- selenium and vitamin E, alone or combined, produce a reduction of the oxidative mechanisms and an improvement of the motility of sperms *(Keskes-Ammar et al, 2003; Scott et al, 1998);*
- vitamin C is capable of improving the count, the motility and the morphology of the sperms *(Akmal et al, 2006);*
- combination of vitamin C and E leads to a reduction of the damage of the DNA *(Greco et al, 2005);*
- combination of L-camitine and acetyl-L-camitine proved efficient in improving the progressive sperm motility, in increasing the total antioxidant capacity of the seminal plasma and in increasing the pregnancy rates in males with OAT-syndrome *(Li et al, 2005; Balercia et al, 2005; Lenzi et al, 2004),* even though a clinically or substantially relevant effect on the motility or on the total sperm count of mobile sperms in males affected by idiopathic asthenospermia is often denied *(Sigman et al, 2006);*

and, regarding other compounds with an adjuvant action:

- folic acid and zinc sulphate, separately, lead to a substantial improvement of the sperm count in subfertile and fertile subjects *(Ebisch et al, 2006; Wong et al, 2002);*
- in vitro addition of L-arginine and polyamines to sperms obtained from males with asthenospermia proved beneficial to the sperm motility *(Morales et al, 2003);*
- vitamin B12, at high doses and with a long term treatment (16 weeks), is of great use in patients affected by oligozoospermia *(Moriyama et al, 1987);*
- treatment of subjects affected by various degrees of oligoastenospermia with arginine opened important perspectives regarding the fertilising capacity of the latter *(Aydin et al, 1995).*
- levels of coenzyme-Q10 in the seminal plasma were correlated to the sperm count and the motility *(Mancini et al, 1994).*

An object of the present invention is a formulation constituted of two different formulations to be administered separately, one containing arginine, vitamin A, vitamin E, vitamin A, vitamin B6, vitamin B9 (i.e. folic acid), vitamin B12, coenzyme Q10 and the other containing carnitine, arginine, vitamin C and zinc. As a matter of fact, such formulation proved useful for the treatment of male infertility, in particular for the treatment of oligoasthenoteratozoospermia (OAT-syndrome).

Preferably, the abovementioned first formulation contains 300 to 500 parts by weight of arginine, 350 to 550 parts by weight of vitamin A, 1 to 3 parts by weight of vitamin B6, 0.0005 to 0.002 parts by weight of vitamin B12, 0.1 to 0.3 parts by weight of vitamin B9, 10 to 30 parts by weight of coenzyme Q10 and 0.07 to 0.009 parts by weight of selenium while said second formulation contains 100 to 300 parts by weight of carnitine, 100 to 300 parts by weight of arginine, 0.05 to 0.15 parts by weight of vitamin C and 10 to 20 parts by weight of zinc. More preferably, said first formulation contains 400 mg of arginine, 450 mg of vitamin A, 30 mg of vitamin E, 2 mg of vitamin B6, 0.001 mg of vitamin B12, 0.2 mg of vitamin B9, 20 mg of coenzyme Q10. 0.082,5 mg of selenium and said second formulation contains 200 mg of carnitine, 200 mg of arginine, 100 mg of vitamin C, 15.2 mg of zinc.

In addition, the formulation of the present invention may further contain common excipients and/or adjuvants such as, for example bulking, anti-caking, bonding and/or colouring agents. In particular, the formulation of the present invention comprises microcrystalline cellulose as a bulking agent, magnesium stearate and/or silicon dioxide as anti-caking agents and chlorophyll and/or curcuma as colouring agents.

The two abovementioned formulations are administered at time intervals in the range of 8 to 14 hours one from the other, preferably in the range of 10 to 12 hours.

In particular, said first formulation is administered in the morning, preferably before breakfast, while the second formulation is administered in the evening, preferably after dinner.

Said formulations are administered for a period of time in the range of 4 to 8 months, preferably for 6 months.

According to a further implementation of the invention, the abovementioned formulation is a combined formulation for simultaneous administration, separated or sequential, capable of substantially improving the sperm quality of patients affected by the OAT-syndrome.

The formulations according to the present invention are preferably indicated for oral administration, more preferably in form of a tablet, capsule or granule.

Further object of the present invention is the use of two different formulations which can be administered separately, the first containing arginine, vitamin A, vitamin E, vitamin B6, vitamin B9 (i.e folic acid), vitamin B12, coenzyme Q10 and the second containing carnitine, arginine, vitamin C and zinc for treating male infertility, in particular for treating idiopathic oligoasthenoteratozoospermia (OAT-syndrome).

In particular, the two abovementioned formulations are administered at a time interval in the range of 8 to 14 hours one from the other, preferably in the range of 10 to 12 hours.

In particular, said first formulation is administered in the morning, preferably before breakfast, while said second formulation is administered in the evening, preferably after dinner.

Said formulations are administered for a period of time in the range of 4 to 8 months, preferably for 6 months.

EXAMPLES

Example 1

Composition in tablet form to be taken every morning, before breakfast

arginine 400 mg
vitamin A 450 mg
vitamin E 30 mg
vitamin B6 2 mg
vitamin B 12 0.0011 mg
vitamin B9 (folic acid) 0.2 mg
coenzyme Q10 20 mg
selenium 0.0825 mg
microcrystalline cellulose 135.52 mg
magnesium stearate 15 mg
silicon dioxide 8 mg
curcuma 5 mg

Example 2:

Composition in tablet form to be taken every evening, after dinner

carnitine 200 mg
arginine 200 mg
vitamin C 100 mg
zinc 15.2 mg
microcrystalline cellulose 106 mg
magnesium stearate 20 mg
silicon dioxide 10 mg
chlorophyll 2 mg

EXPERIMENTAL PART

Various infertile couples were recruited and subjected to the following preliminary tests

- two sperm tests, carried out in the same Laboratory, at a distance of about 2 ½ months one from the other, with a period of sexual abstinence 4-5 days ahead and a complete collection of the ejaculate completed by urethral expression from the perineum to the meatus, during a period of good health of the subject over at least the previous two months. Each sperm sample was examined, calculating its following parameters according to the *WHO - 1999* standards: ejaculate volume (ml), sperm concentration/mm$^3$, total motility (%) at 45', at 120' and at 180' (respectively partitioned into: rapid progressive (%), slow progressive (%), local (%), absent (%)), normal morphology (%).
- the Sperm Index (SI) of each sperm sample was calculated, with the aim of facilitating statistical investigation, according to the following formula:

$$SI = \frac{V \times C \times (\text{mot a} + \sqrt{\text{mot b}}) \times N}{10^{10}}$$

wherein "V" is the ejaculate volume (ml), "C" is the sperm concentration/ml, "mot a" is the rapid progressive motility (%), "mot b" is the slow progressive motility (%), "N" are the normal shapes (%). In brief, the SI attempts to represent in a single numerical value the total count of sperms well mobile and normal-shaped in an ejaculate. For example, an ejaculate having every parameter at its minimum normality value according to *WHO - 1999.* V = 2 ml; C = 20.000.000 sperms/ml; mot a = 25%; mot b = 25%; normal shapes = 15%) has an SI = 1.8, however a truly normal sperm having an SI $\geq$ 5.0.

- In addition, the following additional tests were performed on each semen sample: a) an aniline blue test, to evaluate the condensation and the maturity of the sperm chromatin (v.n.: < 50%) *(Terquem & Dadoune, 1983)*;

  b) the acridine orange test, which calculates the damage to the helices of the DNA of the sperms (v.n.: < 30%) *(Tejada et al, 1984)*;
  c) the swelling test, which identifies the functionally abnormal sperms, that is the ones with a lower fertilising capacity, as well as a higher rate of sperm implantation failure (v.n.: > 60%) *(Check, 2006)*; d) ARIC test, which evaluates the capacity of the sperms to penetrate into the oocytes (v.n.: > 14%.) *(Cummins et al, 1991).*

- Scrotal Colour Doppler, to identify significant pathologies of the testicular pulp, lesions of the epididymis potentially suspected for obstructions and the presence of continuous venous refluxes in orthostatism along the internal spermatic veins.
- Transrectal ultrasonography, to identify significant pathologies of the prostate such as benign prostatic hyperplasia or suspicious cancerous lesions, and suspicious signs for (sub-)obstructions and/or calculosis of the ejaculatory ducts and problems regarding the ampulla-vescicular emptying.
- Karyotype

Furthermore, the following further tests were performed:

- cytology and culture test of the prostatic massage secretion *(Colpi et al, 1982)*, after 7 days of abstinence, only regarding patients whose anamnestic or objective data or whose transrectal ultrasonography data showed or raised suspicions regarding an inflammatory/infective pathology of the prostate.
- plasmatic FSH, LH, Testosterone, Prolactin, Estradiol only for patients with a sperm concentration below 20.000 sperms/mm$^3$.
- Search for Yq microdeletions, only on patients with a sperm concentration below 5.000 nemasperms/mm$^3$.

Inclusion criteria were considered in the present study:

- SI< 5;
- absence of significant pathologies according to Scrotal colour doppler (see above);
- absence of significant pathologies according to transrectal ultrasonography (see above);
- a normal karyotype and a negative search for Yq microdeletions , in cases requiring such test (see above);
- a negative cytology test on the prostatic secretion, or a positive cytology test (but with a negative culture test) after 2 weeks from the end of an antibiotic therapy which had lasted for at least 30 days;
- normal testosterone, prolactin and estradiol, in cases where such tests were required (see above);
- FSH not below the normality range, in cases where its performance was required (see above), in that a low value would have created grounds for a hormonal therapy.

As a whole 40 randomized patients were recruited, to whom the composition of example 1 was administered each morning and the composition of example 2 was administered each evening for a period of 6 months.

At the end of the treatment, all the patients were invited to be subjected to a new sperm test, according to the methods outlined above.

RESULTS

The treatment was well tolerated and there were no symptoms linked to the therapy observed on any of the patients.

The sperm volume increased considerably from 4.07 $\pm$ 1.83 ml (X$\pm$SD) before treatment to 4.55 $\pm$ 1.86 ml after treatment (p < 0.001).

The sperm concentration increased in a very significant manner from 26525 $\pm$ 25251 mm3 (X$\pm$SD) before treatment to 34200 $\pm$ 28254 after treatment (p < 0.0001).

The Total Sperm Motility increased from 47.40 $\pm$ 9.125 % (X$\pm$SD) before treatment to 51.08 $\pm$ 11.711 % after treatment at 45' (p = 0.011), from 44.10 $\pm$ 9.092 % to 47.28 $\pm$ 10.238 % at 120' (p = 0.018), and from 39.95 $\pm$ 9.546 % to 44.90 $\pm$ 11.600 % at 180' (p = 0.003) (Wilcoxon's test).

Sperms with a Normal Morphology rose significantly from 8.18 $\pm$ 4.454 % (X$\pm$SD) before treatment to 10.15 $\pm$ 4.828 % after treatment (p = 0.001) (Wilcoxon's test ).

The Sperm Index improved significantly, passing from 0.820 $\pm$ 1.015 (X$\pm$SD) before treatment to 1.52$\pm$1.760 after treatment (p < 0.001) (two-tail test).

Sperms with positive Swelling Tests (on the 38 patients tested) did not vary significantly, from 66.18 $\pm$ 11.392 % (X$\pm$SD) before treatment to 67.11 $\pm$ 12.944 % after treatment (p = 0.34).

Sperms with positive ARIC Test (on the 34 patients tested) increased in a very significant manner from 7.71 $\pm$ 3.928 % (X$\pm$SD) before treatment to 10.15 $\pm$ 4.279 % after treatment (p < 0.001).

Sperms with chromatin maturation abnormalities detected by the Aniline Blue Test (on the 39 patients tested) did not vary significantly, passing from 48.51 $\pm$ 15.841 % (X$\pm$SD) before treatment to 45.72 $\pm$ 15.970 % after treatment (p = 0.113).

Sperms with DNA abnormalities detected by the acridine orange test (on the 36 patients tested) dropped significantly from 31.03 $\pm$ 13.185 % (X$\pm$SD) before treatment to 25.36 $\pm$ 14.137 after treatment (p = 0.013).

The formulation according to the present invention was therefore effective at significantly improving all the single parameters evaluated in a standard sperm test, also including the Sperm Index, which has the advantage of summarising the total count of well mobile and normally shaped sperms present in an ejaculate in a single numerical value.

Particularly important is the significant improvement induced by the therapy on the percentage of pathological sperms upon the Acridine Orange Test, especially nowadays, where the integrity of the DNA of the sperm is highly considered, with the aim of obtaining a pregnancy at term, both spontaneous and as a result of assisted reproduction techniques.

Equally relevant is the significant improvement of the percentage of sperms positive to the ARIC test, sign of an improved capacity of the sperm to fertilise an oocyte, such data being significant both when attempting to obtain spontaneous fertilisation, and when searching for a correct indication to use intrauterine insemination.

In conclusion, in the field of male infertility therapy, which nowadays shows an almost systematic use of the assisted reproduction techniques and specifically the ICSI, it appears quite interesting to be able to obtain significant sperm improvements using, in patients affected by an actual idiopathic oligoasthenoteratozoospermia (in that preliminarily well selected depending on the possible causal factors of their dispermy), a therapy with vitamins and minerals, such therapy being simple and safe, in that it is substantially free of contraindications.

## Claims

1. Formulation constituted of two different formulations <u>to be administered</u> separately, the first containing arginine, vitamin A, vitamin E, vitamin B6, vitamin B9, vitamin B12, coenzyme Q10 and the second containing carnitine, arginine, vitamin C and zinc, for use in treating male infertility.

2. Formulation for use according to claim 1, wherein said first formulation contains 300 to 500 parts by weight of arginine, 350 to 550 parts by weight of vitamin A, 1 to 3 parts by weight of vitamin B6, 0.0005 to 0.002 parts by weight of vitamin B12, 0.1 to 0.3 parts by weight of vitamin B9, 10 to 30 parts by weight of coenzyme Q10 and 0.07 to 0.09 parts by weight of selenium and said second formulation contains 100 to 300 parts by weight of carnitine, 100 to 300 parts by weight of arginine, 0.05 to 0.15 parts by weight of vitamin C and 10 to 20 parts by weight of zinc.

3. Formulation for use according to claims 1 and 2, wherein said first formulation contains about 400 mg of arginine, about 450 mg of vitamin A, about 30 mg of vitamin E, about 2 mg of vitamin B6, about 0.001 mg of vitamin B12, about 0.2 mg of vitamin B9, about 20 mg of coenzyme Q10 and about 0.0825 mg of selenium and said second formulation contains about 200 mg carnitine, about 200 mg arginine, about 100 mg vitamin C and about 15.2 mg zinc.

4. Formulation for use according to anyone of the preceding claims further containing excipients and/or adjuvants.

5. Formulation for use according to anyone of the preceding claims wherein said formulations are indicated for oral administration.

6. Formulation for use according to anyone of the preceding claims wherein said formulations are in form of a tablet, capsule or granule.

7. Formulation according to claim 1 for use in the treatment of idiopathic oligoasthenoteratazoospermia.

8. Formulation for use according to anyone of the preceding claims wherein said formulations are administered at a time interval in the range of 8 to 14 hours.

9. Formulation for use according to claim 8 wherein said formulations are administered at a time interval in the range of 10 to 12 hours.

10. Formulation for use according to anyone of the preceding claims wherein said first formulation is administered in the morning and said second formulation is administered in the evening.

11. Formulation for use according to anyone of the preceding claims wherein said first formulation is administered before breakfast and said second formulation is administered after dinner.

12. Formulation for use according to anyone of the preceding claims wherein the administration occurs for a period of time in the range of 4 to 8 months.

13. Formulation for use according to claim 12 wherein the administration occurs for a period of time of 6 months.

**Patentansprüche**

1. Formulierung gebildet aus zwei unterschiedlichen Formulierungen, die separat verabreicht werden, die erste enthaltend Arginin, Vitamin A, Vitamin E, Vitamin B6, Vitamin B9, Vitamin B12, Coenzym Q10, und die zweite enthaltend Carnitin, Arginin, Vitamin C und Zink, zur Verwendung in der Behandlung von männlicher Unfruchtbarkeit.

2. Formulierung zur Verwendung gemäß Anspruch 1, wobei die erste Formulierung 300 bis 500 Gewichtsteile Arginin, 350 bis 550 Gewichtsteile Vitamin A, 1 bis 3 Gewichtsteile Vitamin B6, 0,0005 bis 0,002 Gewichtsteile Vitamin B12, 0,1 bis 0,3 Gewichtsteile Vitamin B9, 10 bis 30 Gewichtsteile Coenzym Q10, und 0,07 bis 0,09 Gewichtsteile Selen enthält, und die zweite Formulierung 100 bis 300 Gewichtsteile Carnitin, 100 bis 300 Gewichtsteile Arginin, 0,05 bis 0,15 Gewichtsteile Vitamin C und 10 bis 20 Gewichtsteile Zink enthält.

3. Formulierung zur Verwendung gemäß der Ansprüche 1 und 2, wobei die erste Formulierung ungefähr 400 mg Arginin, ungefähr 450 mg Vitamin A, ungefähr 30 mg Vitamin E, ungefähr 2 mg Vitamin B6, ungefähr 0,001 mg Vitamin B12, ungefähr 0,2 mg Vitamin B9, ungefähr 20 mg Coenzym Q10, und ungefähr 0,0825 mg Selen enthält, und die zweite Formulierung ungefähr 200 mg Carnitin, ungefähr 200 mg Arginin, ungefähr 100 mg Vitamin C und ungefähr 15,2 mg Zink enthält.

4. Formulierung zur Verwendung gemäß einem der vorhergehenden Ansprüche, weiter enthaltend Hilfs- und/oder Zusatzstoffe.

5. Formulierung zur Verwendung gemäß einem der vorhergehenden Ansprüche, wobei die Formulierungen zur oralen Verabreichung angegeben sind.

6. Formulierung zur Verwendung gemäß einem der vorhergehenden Ansprüche, wobei die Formulierungen in Form einer Tablette, Kapsel oder Granulat sind.

7. Formulierung gemäß Anspruch 1 zur Verwendung in der Behandlung von ideopathischer Oligoasthenoteratozoospermie.

8. Formulierung zur Verwendung gemäß einem der vorhergehenden Ansprüche, wobei die Formulierungen in einem Zeitintervall im Bereich von 8 bis 14 Stunden verabreicht werden.

9. Formulierung zur Verwendung gemäß Anspruch 8, wobei die Formulierungen in einem Zeitintervall im Bereich von 10 bis 12 Stunden verabreicht werden.

10. Formulierung zur Verwendung gemäß einem der vorhergehenden Ansprüche, wobei die erste Formulierung am Morgen verabreicht wird und die zweite Formulierung am Abend verabreicht wird.

11. Formulierung zur Verwendung gemäß einem der vorhergehenden Ansprüche, wobei genannte erste Formulierung vor dem Frühstück verabreicht wird und genannte zweite Formulierung nach dem Abendessen verabreicht wird.

12. Formulierung zur Verwendung gemäß einem der vorhergehenden Ansprüche, wobei die Verabreichung für einen Zeitraum im Bereich von 4 bis 8 Monaten stattfindet.

13. Formulierung zur Verwendung gemäß Anspruch 12, wobei die Verabreichung für einen Zeitraum von 6 Monaten stattfindet.

**Revendications**

1. Formulation constituée de deux formulations différentes devant être administrées séparément, la première contenant de l'arginine, de la vitamine A, de la vitamine E, de la vitamine B6, de la vitamine B9, de la vitamine B12, le coenzyme Q10, et la seconde contenant de la carnitine, de l'arginine, de la vitamine C et du zinc, destinée à être utilisée dans le traitement de la stérilité masculine.

2. Formulation destinée à être utilisée selon la revendication 1, où ladite première formulation contient 300 à 500 parties en poids d'arginine, 350 à 550 parties en poids de vitamine A, 1 à 3 parties en poids de vitamine B6, 0,0005 à 0,002 parties en poids de vitamine B12, 0,1 à 0,3 parties en poids de vitamine B9, 10 à 30 parties en poids de coenzyme Q10 et 0,07 à 0,09 parties en poids de sélénium, et ladite seconde formulation contient 100 à 300 parties en poids de carnitine, 100 à 300 parties en poids d'arginine, 0,05 à 0,15 parties en poids de vitamine C et 10 à 20 parties en poids de zinc.

3. Formulation destinée à être utilisée selon les revendications 1 et 2, où ladite première formulation contient environ 400 mg d'arginine, environ 450 mg de vitamine A, environ 30 mg de vitamine E, environ 2 mg de vitamine B6, environ 0,001 mg de vitamine B12, environ 0,2 mg de vitamine B9, environ 20 mg de coenzyme Q10 et environ 0,0825 mg de sélénium, et ladite seconde formulation contient environ 200 mg de carnitine, environ 200 mg d'arginine, environ 100 mg de vitamine C et environ 15,2 mg de zinc.

4. Formulation destinée à être utilisée selon l'une quelconque des revendications précédentes, contenant en outre des excipients et/ou des adjuvants.

5. Formulation destinée à être utilisée selon l'une quelconque des revendications précédentes, où lesdites formulations sont indiquées pour une administration orale.

6. Formulation destinée à être utilisée selon l'une quelconque des revendications précédentes, où lesdites formulations sont sous la forme d'un comprimé, d'une capsule ou d'une granule.

7. Formulation selon la revendication 1, destinée à être utilisée dans le traitement de l'oligo-asthéno-tératozoospermie idiopathique.

8. Formulation destinée à être utilisée selon l'une quelconque des revendications précédentes, où lesdites formulations sont administrées à un intervalle de temps dans la plage de 8 à 14 heures.

9. Formulation destinée à être utilisée selon la revendication 8, où lesdites formulations sont administrées à un intervalle de temps dans la plage de 10 à 12 heures.

10. Formulation destinée à être utilisée selon l'une quelconque des revendications précédentes, où ladite première formulation est administrée le matin et ladite seconde formulation est administrée le soir.

11. Formulation destinée à être utilisée selon l'une quelconque des revendications précédentes, où ladite première formulation est administrée avant le petitdéjeuner et ladite seconde formulation est administrée après le dîner.

12. Formulation destinée à être utilisée selon l'une quelconque des revendications précédentes, où l'administration est réalisée pendant une période de temps dans la plage de 4 à 8 mois.

13. Formulation destinée à être utilisée selon la revendication 12, où l'administration est réalisée pendant une période de temps de 6 mois.